(19) Europäisches Patentamt
European Patent Office
Office européen des brevets

(11) Publication number: 0 237 248 A2

(12) **EUROPEAN PATENT APPLICATION**

(21) Application number: 87301828.7

(22) Date of filing: 03.03.87

(51) Int. Cl.⁴: C07D 495/04 , A61K 31/44 , A61K 31/47 , A61K 31/425 , //(C07D495/04,333:00,235:00)

(30) Priority: 07.03.86 PCT/US86/00496

(43) Date of publication of application: 16.09.87 Bulletin 87/38

(84) Designated Contracting States: AT BE CH DE ES FR GB GR IT LI LU NL SE

(71) Applicant: PFIZER INC.
235 East 42nd Street
New York, N.Y. 10017(US)

(72) Inventor: Lamattina, John Lawrence
13 Huntington Way
Ledyard Connecticut(US)
Inventor: McCarthy, Peter Andrew
2, Box 2141
Pawcatuck Connecticut(US)

(74) Representative: Moore, James William, Dr.
Pfizer Limited Ramsgate Road
Sandwich Kent CT13 9NJ(GB)

(54) **2-[(2-Pyridyl)methylsulfinyl]thienoimidazoles and related compounds as antiulcer agents.**

(57) Antiulcer compounds of the formula

$$R^1-\underset{\underset{O}{\downarrow}}{S}-CH_2-R^2$$

where R¹ is optionally substituted 2-(thieno[3,4-d]-imidazolyl) or 2-(thieno[2,3-d]imidazolyl) and R² is optionally substituted 2-pyridyl, 2-quinolyl or 4-thiazolyl; and corresponding intermediate sulfides, also useful as gastric acid antisecretory agents.

EP 0 237 248 A2

Xerox Copy Centre

# 2-[(2-PYRIDYL)METHYLSULFINYL]THIENOIMIDAZOLES AND RELATED COMPOUNDS AS ANTIULCER AGENTS

The present invention encompasses compounds of the formula

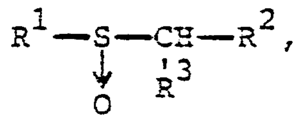

pharmaceutically acceptable salts thereof, and corresponding intermediate sulfides, where $R^1$ is optionally substituted 2-(thieno[3,4-d]imidazolyl) or 2-(thieno[2,3-d]imidazolyl), $R^2$ is optionally substituted 2-pyridyl, 2-quinolyl or 4-thiazolyl, and $R^3$ is hydrogen or methyl. These sulfoxide compounds are $H^+$, $K^+$ ATPase inhibitors <u>in vitro</u>, generally showing gastric antisecretory activity, cytoprotective activity, and inhibition of gastric lesions induced by high doses of piroxicam <u>in vivo</u>. Thus they are useful in mammals, including man, in the treatment or prevention of gastric ulcers; and in combination with piroxicam or a pharmaceutically acceptable salt thereof, provide an improved method for the treatment of inflammation in mammals, including man. The intermediate sulfides are also useful <u>in vivo</u> as inhibitors of gastric acid secretion. This is believed to result from metabolic conversion to the sulfoxide.

Prior art compounds showing $H^+$, $K^+$ ATPase inhibiting activity <u>in vitro</u> as well as gastric antisecretory and cytoprotective activity <u>in vivo</u> include timoprazole, of the formula

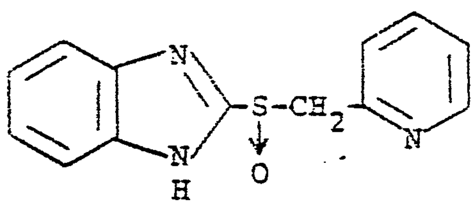

,

omeprazole, of the formula

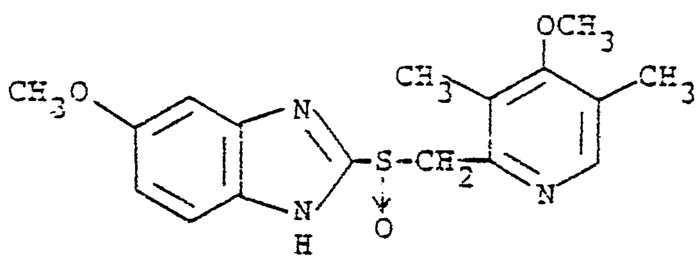

,

and related compounds (see U.S. Patents 4,045,563; 4,045,564; 4,255,431; 4,337,257; 4,359,465 and 4,508,905).

The present invention is directed to sulfoxide compounds of the formula

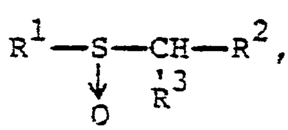

---(I)

wherein
$R^1$ is

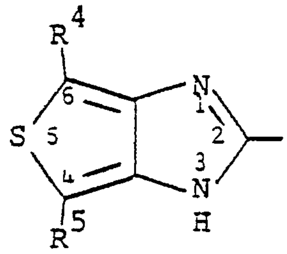

or

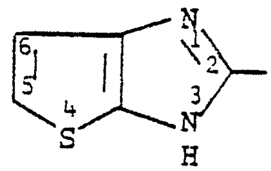

;

$R^2$ is

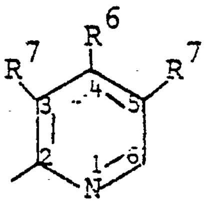

,

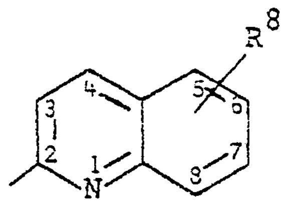

or

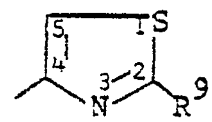

;

$R^3$ is hydrogen or methyl;
$R^4$ and $R^5$ are each independently hydrogen, bromo, chloro, fluoro, $(C_1-C_3)$ alkyl or $[(C_1-C_3)alkoxy]carbonyl$;
$R^6$ is hydrogen or $(C_1-C_3)$ alkoxy;
each $R^7$ is hydrogen or $(C_1-C_3)$ alkyl;
$R^8$ is hydrogen, bromo, chloro, fluoro, methyl or methoxy substituted at the 3, 4, 5, 6, 7 or 8-position; and
$R^9$ is hydrogen, amino, methylamino, dimethylamino or methyl;
and the pharmaceutically acceptable salts thereof.

The bracketed range of carbon atoms refers to the total number of carbon atoms in the alkyl group which follows. The carbon chain can be straight or branched. Because of the better stability of the sulfoxide compounds of the formula (I) in base, pharmaceutically acceptable cationic salts [particularly the $Na^+$, $K^+$, $Ca^{++}$, $Mg^{++}$ or $C(NH_2)_3^+$] are preferred over acid addition salts. However, the sulfides, having relatively good acid stability, are equally useful in the form of their acid addition salts.

Because of its facile preparation and particularly valuable biological properties, the most preferred compound of the present invention is 2-[(2-pyridyl)-methylsulfinyl]thieno[3,4-d]imidazole, the compound of the formula (I) where $R^1$ is thieno[3,4-d]imidazol-2-yl and $R^2$ is 2-pyridyl.

The present invention also encompasses a pharmaceutical composition comprising an antiulcer effective amount of a compound of the formula (I) for use in the treatment or prevention of ulcers in a mammal; a method of treating or preventing ulcers in a mammal which comprises administering to a mammal an antiulcer effective amount of a compound of the formula (I); an antiinflammatory composition comprising an antiinflammatory effective amount of piroxicam or a pharmaceutically acceptable salt thereof and an antiulcer effective amount of a compound of the formula (I); and a method of treating inflammation in a mammal comprising administration to said mammal of an antiinflammatory effective amount of piroxicam or a pharmaceutically acceptable salt thereof and an antiulcer effective amount of a compound of the formula - (I).

Also within the scope of the present invention are sulfide compounds of the formula

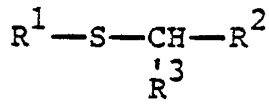

---(II)

and the pharmaceutically acceptable salts thereof where $R^1$, $R^2$ and $R^3$ are as defined above. These compounds are useful as intermediates for the preparation of the sulfoxides of the formula (I), but are also useful as gastric antisecretory agents in their own right.

The compounds of the formula (I) of the present invention are readily prepared by the sequence:

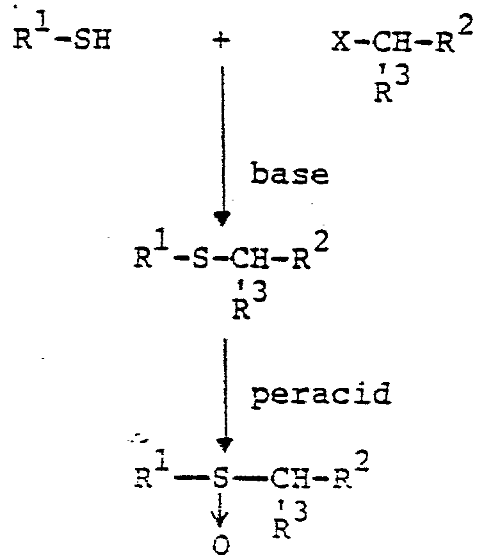


(II)

(I)

where $R^1$, $R^2$ and $R^3$ are as defined above and X is a nucleophilically displaceable group such as chloro, bromo, iodo, mesylate ($OSO_2CH_3$) or tosylate ($OSO_2C_6H_4CH_3$).

The above nucleophilic displacement reaction is facilitated by using an equivalent of a base which is sufficiently strong to convert the mercaptan to its anionic salt, which is more efficient at converting the organic halide or sulfonate ester to the desired sulfide. When an acid addition salt of one of the reactants is employed (e.g., 2-picolyl chloride hydrochloride), an additional compensating amount of base is added. The base is not critical, just so it is of sufficient strength to form the mercaptide anion. In the present case, sodium hydroxide, sodium methoxide or sodium ethoxide are well-suited for the purpose. The present nucleophilic displacement is generally carried out in a solvent. A wide variety of solvents are suitable (e.g., -($C_1$-$C_3$)alcohols, ($C_3$-$C_5$)ketones, acetonitrile or dimethylformamide); just so the reactants have some degree of solubility and are reaction inert (i.e., do not interact with starting materials, intermediates or product in a manner which adversely affects the yield of the desired product). The solvent is preferably less acidic than the mercaptan starting material, so as to facilitate formation of the desired mercaptide anion. Common ethanol is a solvent perfectly well suited for the present purpose. The temperature employed for this reaction is not critical (e.g., 0-120° C.). It should be high enough to provide a reasonable rate, but not so high as to lead to undue side-reactions. As is well known in the art, rate will vary with the nature of the organic halide, the structure of both the halide and the mercaptan, and the solvent. The reaction time should be such that the reaction is nearly complete (e.g., greater than 95% conversion when equivalent amounts of halide and mercaptan are employed) to maximize yields (e.g., 1 hour to several days).

The oxidation of sulfide to sulfoxide is carried out by the action of at least one equivalent of a suitable oxidizing agent, preferably one which is not strongly acidic, in a reaction-inert solvent (as defined above). Suitable oxidizing agents include peracids, m-chloroperbenzoic acid being particularly well suited for this purpose. Suitable solvents include chloroform, methylene chloride, ethanol and ethyl acetate. Over oxidation of the sulfoxide is minimized by use of substantially one equivalent of peracid, e.g., at 0-50° C., or by use of low temperature, e.g., -15 to -45° C., generally with a substantial excess of the peracid, the excess peracid being removed by extraction into aqueous base as the initial stage of workup. The sulfoxide (I) is conveniently then directly isolated as its free base from the organic phase. Methods for preparing the preferred $Na^+$, $K^+$, $Ca^{++}$, $Mg^{++}$ and $[C(NH_2)_3]^+$ (guanidinium) salts are taught in published U.K. Patent Application 2,137,616. Acid addition salts, particularly of the sulfides of the formula (I) are prepared by standard methods which are well known in the art.

The starting materials required for the above sequence, if not commercially available or known in the art, are prepared by conventional methods, as exemplified in specific Preparations below. As a matter of convenience, the sulfur containing starting materials ($R^1$-SH) are herein named as mercapto compounds, e.g., 2-mercaptothieno[3,4-d]imidazole. Of course, those skilled in the art know that said mercaptan is in equilibrium with the thione, e.g., thieno[3,4-d]imidazole-2-thione, and that both yield the same mercaptide:

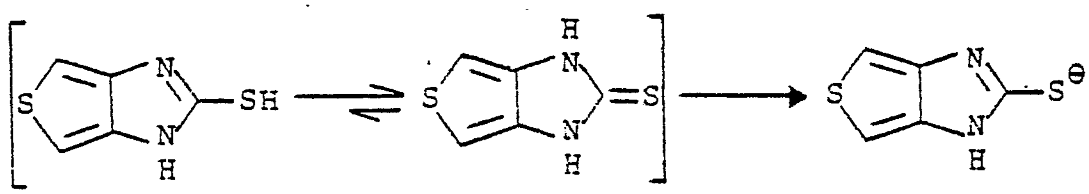

The utility of the present sulfoxide compounds (I) an antiulcer agents is reflected in vitro by their inhibition of $H^+$, $K^+$ ATPase isolated from canine gastric mucosa. The enzyme activity was assayed according to Beil et al., Brit. J. Pharmacol. 82:651-657 (1984) with slight modifications. The enzyme (1-2 micrograms) was preincubated at 37° C. for 45 minutes with a medium containing 2 x $10^{-3}$M $MgCl_2$, 0.05M Tris-Cl buffer (pH 7.5) with or without 0.01M KCl, and the acid activated test drug in a final volume of 0.590 ml. The reaction was started by the addition of 0.010 mmol of ATP (final concentration 3 x $10^{-3}$M). The reaction was terminated by adding trichloroacetic acid to a concentration of 4.2%. Liberated inorganic phosphate was determined using Fiske and Subbarow Reducer available commercially (e.g., from Sigma Chemical Co., P.O. Box 14508, St. Louis, MO 63178, U.S.A.). In this test the drugs are preferably first acid activated by incubating in 1:1 dimethylsulfoxide:0.02N HCl at 37° C. for 30 minutes. In this test preferred 2-[(2-pyridyl)methylsulfinyl]thieno[3,4-b]imidazole showed, without preincubation, an $IC_{50}$ (i.e., concentration which inhibits the enzyme to the extent of 50%) which was 1.2 x $10^{-5}$M when added at the end of the 45 minute preincubation of the enzyme; and 1.0 x $10^{-6}$M when preincubated with the enzyme.

The in vivo utility of the present sulfoxide compounds (I) as antiulcer agents is, in part, reflected by their gastric acid antisecretory activity in dogs, using the method described in Example 6 of U.S. Patent 4,435,396. In this test, preferred 2-[(2-pyridyl)methylsulfinyl]thieno[3,4-b]imidazole showed an $ED_{50}$ (p.o.) of 1.0 mg/kg and an $ED_{50}$ (i.v.) of 0.1 mg/kg. The in vivo utility of the present sulfide compounds as gastric antisecretory agents is also shown by this test.

The in vivo utility of the present sulfoxide compounds (I) an antiulcer agents is particularly shown by their cytoprotective activity. Such activity is demonstrated by the inhibition ethanol induced gastric ulceration in rats, using the method of Example 8 of U.S. Patent 4,435,396. In this test, preferred 2-[(2-pyridyl)methylsulfinyl]thieno[3,4-b]imidazole showed an $ED_{50}$ (p.o.) of 4 mg/kg.

The oral protective effect of the present sulfoxide compounds (I) on piroxicam-induced gastric lesions is determined in rats according to the method of Example 1 of U.S. Patent 4,559,326.

To inhibit (prevent or treat) gastric ulcers in a mammalian subject, the sulfoxide products (I) of the present invention are administered by a variety of conventional routes of administration including orally and parenterally. Preferably, the compounds are administered orally. In general, these compounds will be administered orally at doses between about 0.25 and 50 mg/kg body weight of the mammalian subject to be treated per day, preferably from about 0.5 to 30 mg/kg per day, in single or divided doses. If parenteral administration is desired, then these compounds can be given at total daily doses between about 0.2 and 20 mg/kg body weight of the mammalian subject to be treated. In a 100 Kg man, this translates to a daily oral dosage of about 20-5000 mg/day (preferably about 50-3000 mg/day) and a parenteral dosage of about 20-2000 mg/day. However, at the discretion of the attending physician, some variation in dosage will necessarily occur, depending upon the condition of the subject being treated and the particular compound employed. To inhibit gastric acid secretion, the sulfide compounds (II) are administered to mammals in like manner and quantities.

When co-administering piroxicam and a compound of the formula (I) to a mammal, particularly man, the oral route is preferred. The piroxicam is generally dosed in the range of about 0.1 to 1 mg/kg/day (or about 10-100 mg/day in a 100 Kg man), in single or multiple doses. The compound of the formula (I) is dosed according to the dosage regimen noted above. If desired, the compounds are dosed separately, but they are preferably co-administered in a single, combined formulation suitable for single or multiple daily dosage, as desired. Again, at the discretion of the attending physician, there can be some variation in this dosage regimen.

The sulfoxide compounds of the formula (I) are administered alone or in combination with piroxicam. The sulfide compounds of the formula (II) are generally administered alone. In any case, the active ingredient(s) will generally be further combined with pharmaceutically acceptable carriers or diluents. For oral use, suitable pharmaceutical carriers include inert diluents or fillers, thereby forming dosage forms such as tablets, powders, capsules, and the like. These pharmaceutical compositions can, if desired, contain additional ingredients such as flavorings, binders, excipients and the like. For example, tablets containing various excipients, such as sodium citrate are employed, together with various disintegrants such as starch, alginic acid and certain complex silicates, together with binding agents such as polyvinylpyrrolidone,

sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials therefor include lactose or milk sugar and high molecular weight polyethylene glycols.

For parenteral administration, solutions or suspensions of the compounds of formula (I) in sterile aqueous solutions, for example aqueous propylene glycol, sodium chloride, dextrose or sodium bicarbonate solutions are employed. Such dosage forms are suitably buffered if desired. The preparation of suitable sterile liquid media for parenteral administration will be well known to those skilled in the art.

The present invention is illustrated by the following examples. However, it should be understood that the invention is not limited to the specific details of these examples.

EXAMPLE 1

2-[(2-Pyridyl)methylthio]thieno[3,4-d]imidazole

Sodium hydroxide (11.06 g, 0.276 mol) was added to a mixture of 2-mercaptothieno[3,4-d]imidazole - (20.19 g, 0.129 mol), 2-picolyl chloride hydrochloride (21.21 g, 0.129 mol) and ethanol (400 ml). The resulting suspension was heated to reflux for 3 hours. Decolorizing carbon was cautiously added to the refluxing solution, and the mixture allowed to reflux for 5 minutes, then filtered through diatomaceous earth. The filtrate was concentrated to give crude product as a black oil. Basic alumina chromatography with $CHCl_3$ as eluant followed by trituration with a small amount of chloroform gave 9.87 g (31% yield) of 2-[(2-pyridyl)methylthio]thieno[3,4-d]imidazole of sufficient purity to carry on to the next step. Repetition of the chromatography on impure fractions and trituration procedures gave another 6.27 g of product for a combined yield of 51%. Product prepared in this manner (3.73 g) was recrystallized from acetone to provide 2.83 g of analytically pure material (m.p. 136-137° C., decomposition). Mass spectrum (m/e) : 247 - ($M^+$).

$^1$H-NMR (250 MHz $CDCl_3$)ppm(delta): 4.40 (s, 2H), 6.59 (broad s, IH), 6.86 (broad s, IH), 7.25 (ddd, J = 1.0, 5.0 & 7.0 Hz, IH), 7.38 (dd, J = 1.0 & 7.5 Hz, IH), 7.70 (ddd, J = 1.0, 7.0 & 7.5 Hz, IH), 8.60 (dd, J = 1.0 & 5.0 Hz, IH), 12.17 (s, IH).

$^{13}$C-NMR (63 MHz $CDCl_3$)ppm(delta): 37.4, 92.8, 101.1, 122.8, 123.6, 137.8, 149.0, 157.6, 161.3.

Analysis calculated: C, 53.41; H, 3.68; N, 16.99%.

Found: C, 52.97; H, 3.70; N, 16.73%.

Improvement of this procedure was achieved by running this reaction at room temperature. Thus allowing the reaction to proceed for 8 hours at said temperature led to 20.6 g (65% yield) of title product.

EXAMPLE 2

2-[(2-Pyridyl)methylsulfinyl]thieno[3,4-d]imidazole

A solution of m-chloroperbenzoic acid (13.98 g, 81.0 mmol) in chloroform (910 ml) at room temperature was added dropwise to a -30° C. solution of 2-[(2-pyridyl)methylthio]thieno[3,4-d]imidazole (15.98 g, 64.6 mmol) in chloroform (700 ml) and stirred at -30° C. for 2 hours. At this time, an additional 1.20 g of m-chloroperbenzoic acid was added and the reaction stirred at -30° c. for 1 hour. The cold mixture was washed with saturated sodium bicarbonate (3 x 500 ml). The organic phase was separated, dried with sodium sulfate, dècolorized and filtered over diatomaceous earth to give a bright yellow filtrate. The filtrate was concentrated to a final volume of approximately 100 ml, resulting in precipitation of a yellow powder. Collected, this solid was washed with ether and dried to give 9.63 g (57% yield) of 2-[(2-pyridyl)-methylsulfinyl]thieno[3,4-d]imidazole, (m.p. 150-151° C., decomposition). High resolution mass spectrum - (m/e): calc. 263.0186, found: 263.0166.

$^1$H-NMR (250 MHz, DMSO-$d_6$)ppm(delta): 4.68 (d, J = 12.5 Hz, IH), 4.74 (d, J = 12.5 Hz, IH), 7.02 (broad s, IH), 7.32 (ddd, 1.0, 5.0 & 6.0 Hz, IH), 7.33 (broad s, IH), 7.35 (dd, 1.0 & 8.0 Hz, IH), 7.74 (ddd, 1.0, 6.0 & 8.0 Hz, IH), 8.53 (dd, J = 1.0 & 5.0 Hz, IH), 12.76 (s, IH).

$^{13}$C-NMR (63 MHz, DMSO-$d_6$)ppm(delta): 61.5, 95.1, 104.4, 123.1, 125.3, 136.8, 149.6, 150.6, 163.5.

Analysis calculated:

C, 50.17; H, 3.45; N, 15.96; S, 24.35%.
Found: C, 49.78; H, 3.39; N 15.88; S, 23.95%.
Concentration of the filtrate gave another 2.52 g of desired product for a combined yield of 71%.

EXAMPLE 3

2-[(4-Thiazolyl)methylthio]thieno[3,4-d]imidazole

Using the method of Example 1, 2-mercaptothieno[3,4-d]imidazole (1.20 g, 7.68 mmol) and 4-chloromethylthiazole [1.07 g, 8.06 mmol, prepared according to Caldwell et al., J. Am. Chem. Soc., 73, 2935 (1951)] were coupled. The crude product was triturated three times with ethanol and dried in vacuo to give 0.46 g (24% yield) of title product (m.p. 160°C., decomposition). Mass spectrum (m/e): 253 ($M^+$).
$^1$H-NMR (300 MHz, DMSO-$d_6$)ppm(delta): 4.64 (s, 2H), 6.73 (broad s, IH), 6.97 (broad s, IH), 7.62 (s, IH), 9.02 (s, IH).
$^{13}$C-NMR (75 MHz, DMSO-$d_6$)ppm(delta): 30.4, 92.6, 100.3, 117.2, 152.2, 154.5, 160.1.
Analysis calculated: C, 39.83; H, 3.34; N, 15.48%.
Found: C, 39.92; H, 2.91; N, 15.77%.

EXAMPLE 4

2-[(4-Thiazolyl)methylsulfinyl]thieno[3,4-d]imidazole

Using the method of Example 2, m-chloroperbenzoic acid (0.97 g, 5.62 mmol) was allowed to oxidize 2-[(4-thiazoyl)methylthio]thieno[3,4-d]imidazole (0.95 g, 3.76 mmol). The crude product was purified by basic alumina chromatography using a gradient elution beginning at 2.5% methanol in chloroform and ending at 10% methanol in chloroform. The resulting product was triturated with acetone and dried in vacuo to give 0.30 g (30% yield) of title product as a yellow foam (m.p. 115° C., decomposition). Mass spectrum (m/e): 269 ($M^+$).
$^1$H-NMR (300 MHz, DMSO-$d_6$)ppm(delta): 4.70 (d, J=12 Hz, IH), 4.82 (d, J=12 Hz, IH), 7.20 (s, 2H), 7.68 (s, IH), 9.05 (s, IH).
$^{13}$C-NMR (75 MHz, DMSO-$d_6$)ppm(delta): 55.8, 100.8, 121.3, 145.6, 155.2, 163.4.

PREPARATION 1

2,5-Dibromo-3,4-dinitrothiophene

Concentrated nitric acid (210 ml, 3.23 mol) was added via dropping funnel to a mechanically stirred, 0° C. red solution of 2,5-dibromothiophene (197.77 g, 0.817 mol) in concentrated sulfuric acid (1150 ml, 20.65 mol) under nitrogen. The resulting black solution was stirred at 0° C. for 30 minutes, then cautiously poured onto ice with vigorous mixing by a mechanical stirrer. This mixture was filtered, and the solid washed with water (1000 ml) and dried to give 233.97 g (86% yield) 2,5-dibromo-3,4-dinitrothiophene of sufficient purity to do the next step. Mass spectrum (m/e): 332 ($M^+$). Alternatively, this solid was recrystallized from methanol to give purified product as tan crystals (m.p. 134-137° C., lit. 134-135° C.). Note: For the next step, use of non-crystalline 2,5-dibromo-3,4-dinitrothiophene is advantageous presumably because it dissolves more easily in hydrochloric acid.

PREPARATION 2

3,4-Diaminothiophene Dihydrochloride Stannic Chloride

A few freshly cut pieces of mossy tin were added to a mechanically stirred, 50° C. suspension of crude 2,5-dibromo-3,4-dinitrothiophene (100 g, 0.301 mol) in concentrated hydrochloric acid (2050 ml) under nitrogen. Once the tin dissolved, more tin was added and the mixture cooled to 20° C. using an ice bath. The reaction was kept at 20° C. by adding tin until the full amount (215 g, 1.81 mol) had been added. The

mixture was stirred at room temperature overnight during which time nearly all of the suspended solids dissolved. The mixture was filtered and the filtrate concentrated to a final volume of approximately 900 ml. Crystals were allowed to form overnight at 0° C. These were collected and washed with ethyl ether (600 ml) to obtain 60.79 g (45% yield) of 3,4-diaminothiophene dihydrochloride stannic chloride as a yellow powder (decomposes at 135° C.). Mass spectrum (m/e): 114 ($C_4H_6N_2S+$).
$^1$H-NMR (60 MHz, DMSO-$d_6$)ppm(delta): 7.2 (s, 2H), 8.1 (broad s, 6H).

PREPARATION 3

3,4-Diaminothiophene

To a solution of 3,4-diaminothiophene dihydrochloride stannic chloride (80.01 g, 0.179 mol) in water - (800 ml) was added 20% aqueous sodium hydroxide (approximately 150 ml) until the pH was greater than 12 as judged by pH indicator paper. Ethyl acetate (500 ml) was added and the two phases vigorously mixed, then filtered over diatomaceous earth to remove a flocculant brown precipitate. The aqueous phase was separated, rebasified and extracted twice with ethyl acetate. The combined extracts were dried over sodium sulfate, decolorized with activated carbon, filtered over diatomaceous earth and the filtrate concentrated in vacuo to give 17.13 g (84% yield) of 3,4-diaminothiophene as yellow crystals. This product was carried on to the next step immediately.

PREPARATION 4

2-Mercaptothieno[3,4-d]imidazole (Thieno[3,4-d]imidazole-2-thione)

To a mechanically stirred, 0° C. solution of 3,4-diaminothiophene (17.13 g, 0.150 mol), triethylamine - (62.73 ml, 0.450 mol) and tetrahydrofuran (1300 ml) was added a solution of thiophosgene (11.44 ml, 0.150 mol) in tetrahydrofuran (200 ml) dropwise. After the addition was complete, the mixture was stirred overnight at room temperature. A small amount of precipitate was removed by filtration, and the filtrate decolorized and concentrated to obtain 25.64 g of a brown solid. Trituration of this solid with chloroform provided 20.39 g (87% yield) of 2-mercaptothieno[3,4-d]imidazole as a light tan solid. This material was chromatographically homogenous and pure enough to be carried onto the next step. From another lot, flash chromatography yielded pure 2-mercaptothieno[3,4-d]-imidazole (m.p. greater than 260° C. with discoloration at 170° C., lit. m.p. greater than 300° C.). Mass spectrum (m/e): 156 ($M^+$).
$^1$H-NMR (250 MHz, DMSO-$d_6$)ppm(delta): 6.72 (s, 2H), 12.10 (s, 2H).
$^{13}$C-NMR (63 MHz, DMSO-$d_6$)ppm(delta): 94.3, 135.8, 177.4.
Analysis calculated:
C, 38.44; H, 2.58; N, 17.94; S, 41.04%.
Found: C, 38.41; H, 2.72; N, 17.86; S, 40.44%.

## Claims

1. A compound of the formula

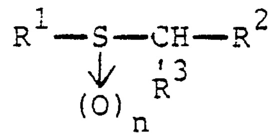 (I)

wherein n is 0 or 1;

$R^1$ is

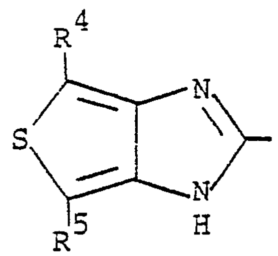 or 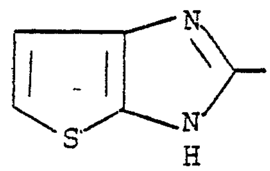 ;

$R^2$ is

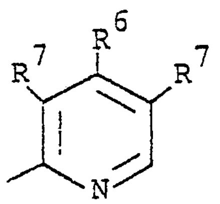 , 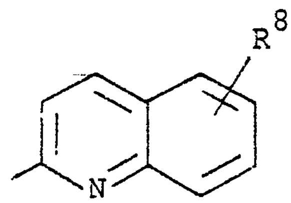 or 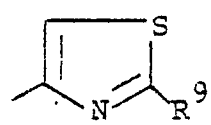 ;

$R^3$ is hydrogen or methyl;
$R^4$ and $R^5$ are each independently hydrogen, bromo, chloro, fluoro, $(C_1\text{-}C_3)$alkyl or $[(C_1\text{-}C_3)$alkoxy]carbonyl;
$R^6$ is hydrogen or $(C_1\text{-}C_3)$alkoxy;
each $R^7$ is hydrogen or $(C_1\text{-}C_3)$alkyl;
$R^8$ is hydrogen, bromo, chloro, fluoro, methyl or methoxy; and
$R^9$ is hydrogen, amino, methylamino, dimethylamino or methyl;
or a pharmaceutically acceptable salt thereof.

2. A compound of claim 1 wherein n is 1.

3. A compound of claim 2 wherein
$R^1$ is

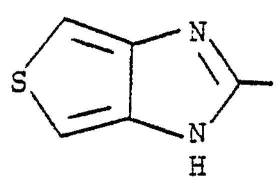 ;

$R^2$ is

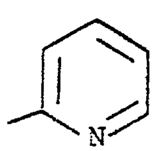 ; and

$R^3$ is hydrogen.

4. A compound of claim 1 wherein n is 0.

5. The compound of claim 4 wherein $R^1$ is

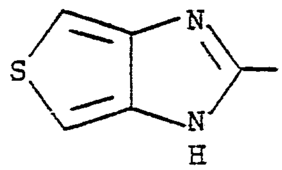

;

$R^2$ is

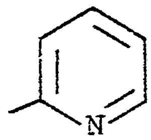

; and

$R^3$ is hydrogen.

6. A pharmaceutical composition comprising a compound of the formula I as claimed in claim 2 or claim 3, together with a pharmaceutically acceptable diluent or carrier.

7. A pharmaceutical composition as claimed in claim 6 which further includes an antiinflammatory effective amount of piroxicam or a pharmaceutically acceptable salt thereof.

8. A compound of the formula (I) as claimed in claim 2 or claim 3 for use in medicine in particular for the treatment of gastric ulcers.

CLAIMS FOR THE FOLLOWING CONTRACTING STATES: AT, ES

1. A process for a compound of the formula

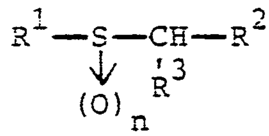

(I)

wherein n is 0 or 1;

$R^1$ is

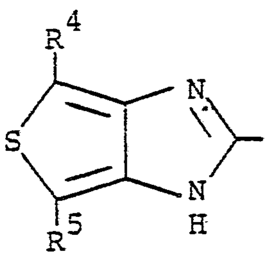

or

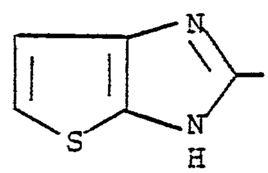

;

$R^2$ is

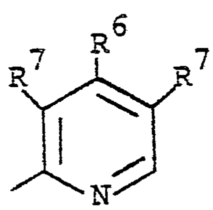

,

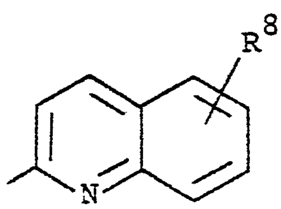

or

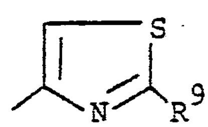

;

$R^3$ is hydrogen or methyl;

$R^4$ and $R^5$ are each independently hydrogen, bromo, chloro, fluoro, $(C_1-C_3)$alkyl or $[(C_1-C_3)$alkoxy]carbonyl;

$R^6$ is hydrogen or $(C_1-C_3)$alkoxy;

each $R^7$ is hydrogen or $(C_1-C_3)$alkyl;

$R^8$ is hydrogen, bromo, chloro, fluoro, methyl or methoxy; and

$R^9$ is hydrogen, amino, methylamino, dimethylamino or methyl;

or a pharmaceutically acceptable salt thereof;

which comprises, when n = 0, reaction of a compound of the formula

$R^1SH$,

where $R^1$ is as defined above, with a compound of the formula

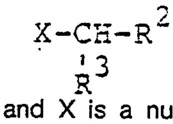 ,

where $R^2$ and $R^3$ are as defined above, and X is a nucleophilically displaceable group such as chloro, bromo, iodo, mesylate or tosylate, in the presence of a base of sufficient strength to convert the mercaptan to its anionic salt in a reaction-inert solvent; and when n is 1, oxidation of a preformed compound of the formula (I) wherein n is 0 with a peracid in a reaction-inert solvent.

2. A process of claim 1 for a compound wherein n is 1.

3. The process for a compound of claim 2 wherein $R^1$ is

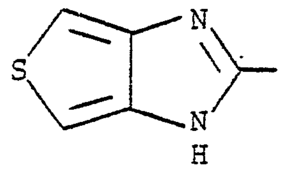 ;

$R^2$ is

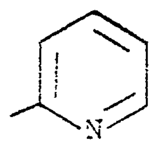 ; and

$R^3$ is hydrogen.

4. A process of claim 1 for a compound wherein n is 0.

5. The process for a compound of claim 4 wherein $R^1$ is

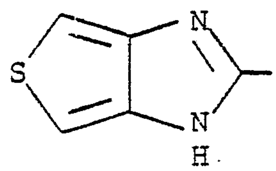 ;

$R^2$ is

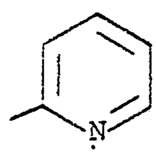 ; and

$R^3$ is hydrogen.

CLAIMS FOR THE FOLLOWING CONTRACTING STATE : GR

1. A process for a compound of the formula

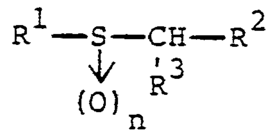

(I)

wherein n is 0 or 1;

$R^1$ is

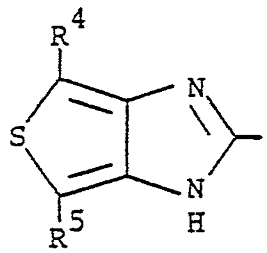

or

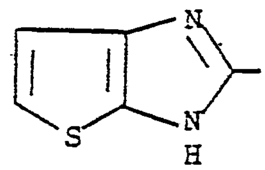

;

$R^2$ is

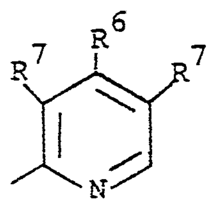

,

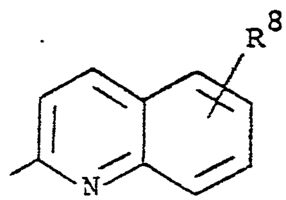

or

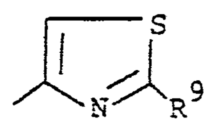

;

$R^3$ is hydrogen or methyl;
$R^4$ and $R^5$ are each independently hydrogen, bromo, chloro, fluoro, $(C_1$-$C_3)$alkyl or $[(C_1$-$C_3)$alkoxy]carbonyl;
$R^6$ is hydrogen or $(C_1$-$C_3)$alkoxy;
each $R^7$ is hydrogen or $(C_1$-$C_3)$alkyl;
$R^8$ is hydrogen, bromo, chloro, fluoro, methyl or methoxy; and
$R^9$ is hydrogen, amino, methylamino, dimethylamino or methyl;
or a pharmaceutically acceptable salt thereof;
which comprises, when n = 0, reaction of a compound of the formula
$R^1SH$,
where $R^1$ is as defined above, with a compound of the formula

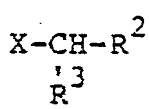

,

where $R^2$ and $R^3$ are as defined above, and X is a nucleophilically displaceable group such as chloro, bromo, iodo, mesylate or tosylate, in the presence of a base of sufficient strength to convert the mercaptan to its anionic salt in a reaction-inert solvent; and when n is 1, oxidation of a preformed compound of the formula (I) wherein n is 0 with a peracid in a reaction-inert solvent.

2. A process of claim 1 for a compound wherein n is 1.

3. The process for a compound of claim 2 wherein $R^1$ is

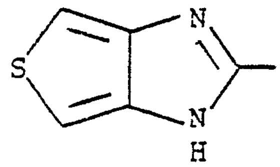

;

$R^2$ is

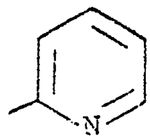

; and

$R^3$ is hydrogen.

4. A process of claim 1 for a compound wherein n is 0.

5. The process for a compound of claim 4 wherein $R^1$ is

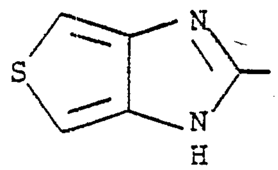

;

$R^2$ is

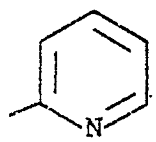

; and

$R^3$ is hydrogen.

6. A compound of the formula (I) as defined in claim 1 wherein n is 0.